# EUROPEAN PATENT SPECIFICATION

(11) **EP 3 745 949 B1**
(45) Date of publication and mention of the grant of the patent: **20.08.2025**
(21) Application number: 19714857.0
(22) Date of filing: 31.01.2019
(51) Int. Cl.: A61B 5/00, G16Z 99/00

(54) **A MONITORING SYSTEM, A METHOD AND A COMPUTER PROGRAM PRODUCT**
ÜBERWACHUNGSSYSTEM, VERFAHREN UND COMPUTERPROGRAMMPRODUKT
SYSTÈME DE SURVEILLANCE, PROCÉDÉ ET PRODUIT PROGRAMME D'ORDINATEUR

(30) Priority: 31.01.2018 NL 2020351
(43) Date of publication of application: 09.12.2020
(73) Proprietor: Sensara Group B.V., 3069 NJ Rotterdam (NL)
(72) Inventor: KARKOWSKI, Ireneusz Piotr, 2622 KM Delft (NL)
(74) Representative: V.O.
(86) International application number: PCT/NL2019/050060
(87) International publication number: WO 2019/151857

(56) References cited:
- EP-A2- 1 571 583
- EP-A2- 1 571 583
- US-A1- 2007 162 304
- US-A1- 2007 162 304
- US-A1- 2016 027 278
- ANDREA KEALY ET AL: "Derivation of night time behaviour metrics using ambient sensors", PERVASIVE COMPUTING TECHNOLOGIES FOR HEALTHCARE (PERVASIVEHEALTH), 2013 7TH INTERNATIONAL CONFERENCE ON, IEEE, 5 May 2013 (2013-05-05), pages 33 - 40, XP032439570, ISBN: 978-1-4799-0296-5, DOI: 10.4108/ICST.PERVASIVEHEALTH.2013.252095
- ANDREA KEALY ET AL: "Derivation of night time behaviour metrics using ambient sensors", PERVASIVE COMPUTING TECHNOLOGIES FOR HEALTHCARE (PERVASIVEHEALTH), 2013 7TH INTERNATIONAL CONFERENCE ON, IEEE, 5 May 2013 (2013-05-05), pages 33 - 40, XP032439570, ISBN: 978-1-4799-0296-5, DOI: 10.4108/ICST.PERVASIVEHEALTH.2013.252095

## Description

The invention relates to a monitoring system for monitoring human activities, comprising a multiple number of presence detection sensors mounted in a residence, further comprising a control unit communicately connected to the presence detection sensors and arranged for receiving presence data from the presence detection sensors.

Monitoring systems including sensors and a control unit are e.g. known from patent publication US 2017/0078298 and can be used to secure premises, such as an office, a warehouse, a factory and other public or private premises. Further, monitoring systems are known for monitoring human activities, e.g. for tracking a presence of persons in a building, as e.g. disclosed in European patent EP 2 353 153 B1 mentioning the same inventor.

It is an object of the present invention to provide an improved system for monitoring human activities. Thereto, according to the invention, a monitoring system according to claim 1 is provided, wherein the control unit is further arranged for determining, from the received presence data, whether the inhabitant of the residence stays indoors or outdoors and for determining a time interval of an outdoor stay.

By determining whether the inhabitant of the residence stays indoors or outdoors and by determining a time interval of an outdoor stay, relevant information about human activities of the resident can be obtained in a relatively easy way.

The invention is at least partly based on the insight that by specifically interpreting presence detection sensors mounted in a residence, highly relevant data about outdoor stay of the inhabitant can be obtained in relatively easy, cheap though accurate way. Further, outdoor stay data can be processed for evaluating social interaction behaviour of the inhabitant, e.g. for detection of a trend in social isolation that may be early indicator of dementia.

It is noted that patent publication US 2016/0027278 A1 discloses a system for event-based monitoring of a subject's well-being within an unattended setting. A plurality of sensors are disposed within the setting for sensing disparate events.

The invention also relates to a method of monitoring human activities according to claim 6.

Further, the invention relates to a computer program product according to claim 7. A computer program product may comprise a set of computer executable instructions stored on a data carrier, such as a CD or a DVD. The set of computer executable instructions, which allow a programmable computer to carry out the method as defined above, may also be available for downloading from a remote server, for example via the Internet.

Further advantageous embodiments according to the invention are described in the following claims.

It should be noted that the technical features described above or below may each on its own be embodied in a system or method, i.e. isolated from the context in which it is described, separate from other features, or in combination with only a number of the other features described in the context in which it is disclosed. Each of these features may further be combined with any other feature disclosed, in any combination.

The invention will now be further elucidated on the basis of a number of exemplary embodiments and an accompanying drawing. In the drawing:
Fig. 1 shows a schematic view of a monitoring system according to the invention, and
Fig. 2 shows a flow chart of a method according to the invention.

It is noted that the figures show merely preferred embodiments according to the invention. In the figures, the same reference numbers refer to equal or corresponding parts.

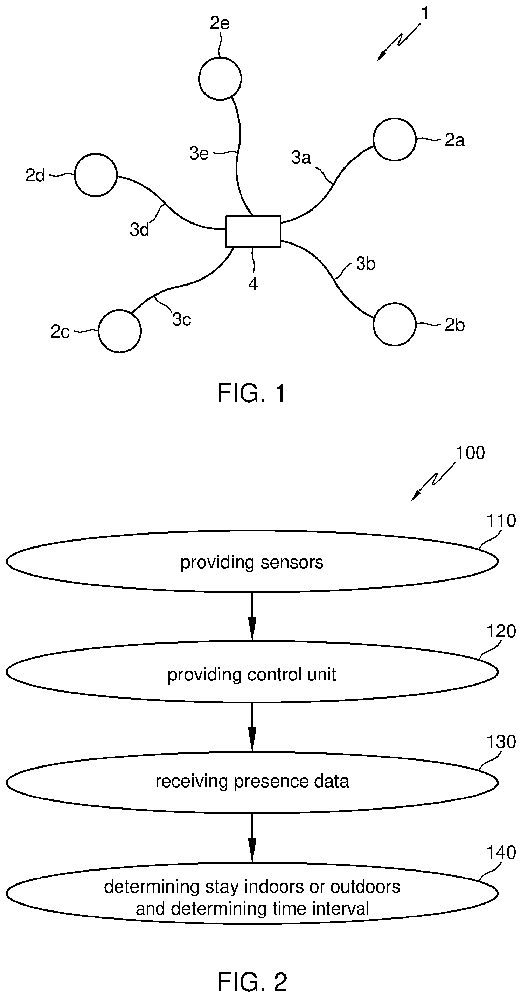

Figure 1 shows a schematic view of a monitoring system 1 according to the invention. The system 1 is arranged for monitoring human activities, and comprises a multiple number of presence detection sensors 2a-e mounted in a residence of a user of the system 1. Further, the system 1 comprises a multiple number of communication lines 3a-e, each the communication lines 3a-d being connected, at a first end, to a respective presence detection sensor. The system 1 also has a control unit 4 that is connected to a second end, opposite to the first end, of the communication lines 3a-e for communicately connecting the control unit 4 to the presence detection sensors 2a-e. The control unit 4 is arranged for receiving presence data from the presence detection sensors 2a-e, via said communication lines 3a-d.

In the embodiment shown in Fig. 1, the sensors 2 and the control unit 4 are communicately connected via a star-shaped structure. However, the communication structure can be implemented in various other ways, e.g. by using a ring-shaped structure. Further, wired or wireless communication channels can be applied.

The presence detection sensors 2 include a single or a multiple number of door sensors, and, optionally, infrared motion sensors, other activity sensors and/or occupancy sensors.

During operation of the system 1, the presence detection sensors 2 sense a physical parameter, e.g. infrared signals, and transmit said sensed parameter as presence data towards the control unit 4. The sensors 2 may operate in a continuous manner or intermittently e.g. at regular time intervals. Further, the sensors may transmit the presence data immediately upon sensing or at a later instant, e.g. at regular time intervals.

The control unit 4 is arranged for determining, from the received presence data, whether a person living in the residence, also referred to as the inhabitant of the residence stays indoors or outdoors. Also, the control unit 4 is arranged for determining a time interval of an outdoor stay.

The control unit 4 can be implemented as a single integrated unit, as shown in Fig. 1. Alternatively, the control unit 4 is composed of two or more separate units, in a distributed manner. As an example, the control unit 4 includes a residence module for receiving the presence data from the sensors and a central unit located at a central location in the residence or remotely outdoors for performing the step of determining whether the inhabitant is indoors or outdoors and of determining a time interval of an outdoor stay. The residence module is connected to the central unit, e.g. via Internet or another network such as a local are network, LAN, for transmitting the received presence data. Optionally, the central unit is connected to a multiple number of residence modules for monitoring human activities in respective residences.

Further, the control unit 4 may be arranged for transmitting further signals, based on the determined data regarding outdoor stay of the inhabitant.

The control unit 4 determines that the inhabitant of the residence stays outdoors by detecting that a front door has opened and closed and that, after the door has closed, no activities have been detected anymore. Similarly, the control unit 4 determines that the inhabitant enters the residence, and is thus indoors, after the front door has opened and closed, after an outdoor time period, and activities of the user indoors are sensed.

Further, the time interval of an outdoor stay is determined by detecting, from the received presence data, an inhabitant's departure from and an inhabitant's arrival to the residence.

The control unit 4 is further arranged for counting a number of outdoor stays having a time interval that is longer than a predetermined threshold time period. As an example, a number of outdoor stays longer than circa three hours can be counted, e.g. per day, week or another time period. As another example, the predetermined threshold time period can be less than three hours, e.g. two hours, or more than three hours, e.g. four hours. If an outdoor stay lasts longer than the predetermined threshold time period, it is assumed that the inhabitant contributes in a social event such as an outdoor visit.

The control unit 4 is further arranged for counting a number of home visits, by non-inhabitant visitors, in the residence of the inhabitant. The home visits are counted by comparing detection data of the front door being opened and closed with multiple simultaneous activities sensed in the residence, e.g. at separate locations in the residence.

Also, the control unit 4 is arranged for determining a social interaction indicator SII based on a total number of counted outdoor stays OS, i.e. the outdoor stays that have a time interval longer than the predetermined threshold time period, and/or counted home visits HV in an evaluation time period. As an example, the evaluation time period is a week so that a total number of counted outdoor stays OS and counted home visits HV in a week can be determined. Preferably, the total number of counted outdoor stays OS are added to the counted home visits HV in a weighted manner, e.g. by multiplying the counted outdoor stays OS with a first weighing factor f1 and the counted home visits HV by a weighing factor f2, before adding. Alternatively, the total number of counted outdoor stays OS and counted home visits HV are just added, or the social interaction indicator SII is calculated by multiplying the number of counted outdoor stays OS by the number of counted home visits HV. In principle, the social interaction indicator SII may be based on the number of counted outdoor stays OS only, or may be based on the number of counted home visits HV only.

Preferably, the control unit 4 is arranged for determining the social interaction indicator SII in subsequent evaluation time periods, such that an evolution of the social interaction indicator SII can be monitored. In another embodiment, the social interaction indicator SII is not determined in subsequent evaluation time periods, but over other evaluation time periods, e.g. at predetermined intervals or upon a request of a user of the system 1.

The control unit 4 can be arranged for transmitting further signals, based on the determined social interaction indicator SII or on a time dependent evolution of the determined social interaction indicator SII over time. The control unit 4 sends an information signal to a software platform, e.g. via a communication channel connecting the control unit 4 to a device on which said software platform is running, such as a local device having Internet connectivity or access to telephone services, informing relatives and/or professional caregivers about a trend in social interaction indicator values of the resident, e.g. via a software service such as an app. Advantageously, such a trend in social interaction can thus be detected automatically and in a privacy friendly manner, as specific data on individual entering and leaving events can be filtered out. By informing a relative and/or professional caregiver about any negative/decreasing social interaction trend in an early stage, preventive measures can be taken to the resident.

Optionally, the control unit 4 can be arranged for informing the resident that an information signal has been transmitted to a relative and/or professional caregiver, e.g. via an optical indicator.

Figure 2 shows a flow chart of a method according to the invention. The method is used for monitoring human activities. The method 100 comprises a step of providing 110 a multiple number of presence detection sensors mounted in a residence, a step of providing 120 a control unit communicately connected to the presence detection sensors, a step of receiving 130 presence data from the presence detection sensors, and a step of determining 140, from the received presence data, whether the inhabitant of the residence stays indoors or outdoors and determining a time interval of an outdoor stay.

The step of receiving presence data from the presence detection sensors, and the step of determining, from the received presence data, whether the inhabitant of the residence stays indoors or outdoors and determining a time interval of an outdoor stay can be performed using dedicated hardware structures, such as FPGA and/or ASIC components. Otherwise, the method can at least partially be performed using a computer program product comprising instructions for causing a processor of a computer system to perform the above described steps. A number of steps can in principle be performed on a single processor. However it is noted that at least one step can be performed on a separate processor, e.g. the step of receiving presence data.

The invention is not restricted to the embodiments described above. It will be understood that many variants are possible.

These and other embodiments will be apparent for the person skilled in the art and are considered to fall within the scope of the invention as defined in the following claims. For the purpose of clarity and a concise description features are described herein as part of the same or separate embodiments. However, it will be appreciated that the scope of the invention may include embodiments having combinations of all or some of the features described.

## Claims

1. A monitoring system (1) for monitoring human activities, comprising a multiple number of presence detection sensors (2a-e) mounted in a residence, further comprising a control unit (4) communicately connected to the presence detection sensors (2a-e) and arranged for receiving presence data from the presence detection sensors (2a-e), wherein the control unit (4) further is arranged for determining, from the received presence data, whether the inhabitant of the residence stays indoors or outdoors and for determining a time interval of an outdoor stay, **characterized in that** the presence detection sensors (2a-e) include a single or a multiple number of door sensors,
wherein the control unit (4) is further arranged for counting a number of outdoor stays having a time interval that is longer than a predetermined threshold time period,
wherein the control unit (4) is arranged for determining that the inhabitant of the residence stays outdoors by detecting that a front door has opened and closed and that, after the door has closed, no activities have been detected anymore,
wherein the control units (4) is arranged for determining that the inhabitant enters the residence, and is thus indoors, by detecting that the front door has opened and closed, after an outdoor time period, and activities of the user indoors are sensed,
wherein the control unit (4) is further arranged for counting a number of home visits, by non-inhabitant visitors, in the residence of the inhabitant by comparing detection data of the front door being opened and closed with multiple simultaneous activities sensed in the residence,
wherein the control unit (4) is arranged for determining a social interaction indicator based on a total number of counted outdoor stays and/or counted home visits in an evaluation time period, and
wherein the control unit (4) is also arranged for sending an information signal to a software platform informing relatives and/or professional caregivers, based on the determined social interaction indicator or on a time dependent evolution of the determined social interaction indicator.

2. A monitoring system according to claim 1, wherein the time interval of an outdoor stay is determined by detecting, from the received presence data, an inhabitant's departure from and an inhabitant's arrival to the residence.

3. A monitoring system according to claim 1 or 2, wherein the presence detection sensors include a single or a multiple number of infrared motion sensors and/or occupancy sensors.

4. A monitoring system according to any of the preceding claims, wherein the total number of counted outdoor stays are added to the counted home visits in a weighted manner.

5. A monitoring system according to any of the preceding claims, wherein the control unit is arranged for determining the social interaction indicator in subsequent evaluation time periods.

6. A method of monitoring human activities, comprising the steps of:
- providing a multiple number of presence detection sensors (2a-e) including a single or a multiple number of door sensors mounted in a residence;
- providing a control unit (4) communicately connected to the presence detection sensors (2a-e);
- receiving presence data from the presence detection sensors (2a-e);
- determining, from the received presence data, whether the inhabitant of the residence stays indoors or outdoors and determining a time interval of an outdoor stay,
wherein the determination that the inhabitant of the residence stays outdoors is performed by detecting that a front door has opened and closed and that, after the door has closed, no activities have been detected anymore,
wherein the determination that the inhabitant enters the residence, and is thus indoors, is performed by detecting that the front door has opened and closed, after an outdoor time period, and activities of the user indoors are sensed;
- counting a number of outdoor stays having a time interval that is longer than a predetermined threshold time period, and/or
- counting a number of home visits, by non-inhabitant visitors, in the residence of the inhabitant by comparing detection data of the front door being opened and closed with multiple simultaneous activities sensed in the residence,
- determining a social interaction indicator based on a total number of counted outdoor stays and/or counted home visits in an evaluation time period, and
- sending an information signal to a software platform informing relatives and/or professional caregivers, based on the determined social interaction indicator or on a time dependent evolution of the determined social interaction indicator.

7. A computer program product for monitoring human activities, the computer program product comprising computer readable code for causing a processor to perform a step of receiving presence data from a multiple number of presence detection sensors (2a-e) including a single or a multiple number of door sensors, mounted in a residence and communicately connected to a control unit (4), a step of determining, from the received presence data, whether the inhabitant of the residence stays indoors or outdoors and for determining a time interval of an outdoor stay,
wherein the determination that the inhabitant of the residence stays outdoors is performed by detecting that a front door has opened and closed and that, after the door has closed, no activities have been detected anymore,
wherein the determination that the inhabitant enters the residence, and is thus indoors, is performed by detecting that the front door has opened and closed, after an outdoor time period, and activities of the user indoors are sensed,
a step of counting a number of outdoor stays having a time interval that is longer than a predetermined threshold time period, and/or a step of counting a number of home visits, by non-inhabitant visitors, in the residence of the inhabitant,
wherein the home visits are counted by comparing detection data of the front door being opened and closed with multiple simultaneous activities sensed in the residence,
a step of determining a social interaction indicator based on a total number of counted outdoor stays and/or counted home visits in an evaluation time period, and a step of sending an information signal to a software platform informing relatives and/or professional caregivers, based on the determined social interaction indicator or on a time dependent evolution of the determined social interaction indicator.

## Patentansprüche

1. Überwachungssystem (1) zum Überwachen menschlicher Aktivitäten, umfassend eine Mehrzahl an in einer Wohnstätte montierten Anwesenheitserkennungssensoren (2a-e), ferner umfassend eine Steuereinheit (4), kommunikativ verbunden mit den Anwesenheitserkennungssensoren (2a-e) und angeordnet zum Empfangen von Anwesenheitsdaten von den Anwesenheitserkennungssensoren (2a-e), wobei die Steuereinheit (4) ferner dazu angeordnet ist, aus den empfangenen Anwesenheitsdaten festzustellen, ob sich der Bewohner der Wohnstätte innerhalb oder außerhalb aufhält, und um ein Zeitintervalls eines Aufenthalts außerhalb festzustellen, **dadurch gekennzeichnet, dass** die Anwesenheitserkennungssensoren (2a-e) einen einzelnen oder eine Mehrzahl an Türsensoren umfassen,
wobei die Steuereinheit (4) ferner zum Zählen einer Anzahl von Aufenthalten außerhalb mit einem Zeitintervall angeordnet ist, das länger als ein vorbestimmter Schwellenzeitraum ist,
wobei die Steuereinheit (4) dazu angeordnet ist, festzustellen, dass sich der Bewohner der Wohnstätte außerhalb aufhält, indem sie erkennt, dass eine Eingangstür geöffnet und geschlossen wurde und dass nach dem Schließen der Tür keine Aktivitäten mehr erkannt wurden,
wobei die Steuereinheiten (4) dazu angeordnet sind, festzustellen, dass der Bewohner die Wohnstätte betritt und sich somit innerhalb befindet, indem sie erkennen, dass die Eingangstür nach einer Zeitdauer außerhalb geöffnet und geschlossen wurde, und Aktivitäten des Benutzers innerhalb erfasst werden,
wobei die Steuereinheit (4) ferner zum Zählen einer Anzahl an häuslichen Besuchen in der Wohnstätte des Bewohners durch nicht in der Wohnstätte wohnende Besucher durch Vergleichen von Daten der Erkennung des geöffneten und geschlossenen Zustands der Eingangstür mit mehreren gleichzeitigen, in der Wohnstätte erfassten Aktivitäten angeordnet ist,
wobei die Steuereinheit (4) zum Ermitteln eines Indikators für soziale Interaktion basierend auf einer Gesamtzahl gezählter Aufenthalte außerhalb und/oder gezählter häuslicher Besuche der Wohnstätte in einem Auswertungszeitraum angeordnet ist, und
wobei die Steuereinheit (4) auch zum Senden eines Informationssignals an eine Softwareplattform angeordnet ist, informierend Angehörige und/oder professionelle Betreuer, basierend auf dem ermittelten Indikator für soziale Interaktion oder auf einer zeitabhängigen Entwicklung des ermittelten Indikators für soziale Interaktion.

2. Überwachungssystem nach Anspruch 1, wobei das Zeitintervall eines Aufenthalts außerhalb dadurch bestimmt wird, dass aus den empfangenen Anwesenheitsdaten der Weggang eines Bewohners aus und die Ankunft eines Bewohners in der Wohnstätte festgestellt wird.

3. Überwachungssystem nach Anspruch 1 oder 2, wobei die Anwesenheitserkennungssensoren einen einzelnen oder eine Mehrzahl an Infrarotbewegungssensoren und/oder Anwesenheitssensoren umfassen.

4. Überwachungssystem nach einem der vorhergehenden Ansprüche, wobei die Gesamtzahl der gezählten Aufenthalte außerhalb zu den gezählten häuslichen Besuchen gewichtet addiert wird.

5. Überwachungssystem nach einem der vorhergehenden Ansprüche, wobei die Steuereinheit zum Ermitteln des Indikators für soziale Interaktion in nachfolgenden Auswertungszeiträumen angeordnet ist.

6. Verfahren zum Überwachen menschlicher Aktivitäten, umfassend die Schritte:
- Bereitstellen einer Mehrzahl an Anwesenheitserkennungssensoren (2a-e), einschließlich eines einzelnen oder einer Mehrzahl an Türsensoren, montiert in einer Wohnstätte;
- Bereitstellen einer Steuereinheit (4), die mit den Anwesenheitserkennungssensoren (2a-e) kommunikativ verbunden ist;
- Empfangen von Anwesenheitsdaten von den Anwesenheitserkennungssensoren (2a-e);
- Feststellen, aus den empfangenen Anwesenheitsdaten, ob sich der Bewohner der Wohnstätte innerhalb oder außerhalb aufhält, und Feststellen eines Zeitintervalls eines Aufenthalts außerhalb,
wobei die Feststellung, dass sich der Bewohner der Wohnstätte außerhalb aufhält, dadurch erfolgt, dass erkannt wird, dass eine Eingangstür geöffnet und geschlossen wurde und dass nach dem Schließen der Tür keine Aktivitäten mehr erkannt wurden,
wobei die Feststellung, dass der Bewohner die Wohnstätte betritt und sich somit innerhalb befindet, dadurch erfolgt, dass erkannt wird, dass die Eingangstür nach einer Zeitdauer außerhalb geöffnet und geschlossen wurde, und Aktivitäten des Benutzers innerhalb erfasst werden;
- Zählen einer Anzahl von Aufenthalten außerhalb mit einem Zeitintervall, das länger als ein vorbestimmter Schwellenzeitraum ist und/oder
- Zählen einer Anzahl von häuslichen Besuchen der Wohnstätte durch nicht in der Wohnstätte des Bewohners wohnende Besucher durch Vergleichen der Erkennungsdaten der geöffneten und geschlossenen Eingangstür mit mehreren gleichzeitigen Aktivitäten, die in der Wohnstätte erfasst wurden,
- Ermitteln eines Indikators für soziale Interaktion basierend auf einer Gesamtzahl gezählter Aufenthalte außerhalb und/oder gezählter häuslicher Besuche in der Wohnstätte in einem Auswertungszeitraum, und
- Senden eines Informationssignals an eine Softwareplattform, wodurch Angehörige und/oder professionelle Betreuer basierend auf dem ermittelten Indikator für soziale Interaktion oder einer zeitabhängigen Entwicklung des ermittelten Indikators für soziale Interaktion informiert werden.

7. Computerprogrammprodukt zum Überwachen menschlicher Aktivitäten, wobei das Computerprogrammprodukt computerlesbaren Code umfasst, um einen Prozessor zu veranlassen, einen Schritt des Empfangens von Anwesenheitsdaten von einer Mehrzahl an Anwesenheitserkennungssensoren (2a-e), umfassend einen einzelnen oder eine Mehrzahl an Türsensoren, montiert in einer Wohnstätte und kommunikativ mit einer Steuereinheit (4) verbunden, einen Schritt des Feststellens, aus den empfangenen Anwesenheitsdaten, ob sich der Bewohner der Wohnstätte innerhalb oder außerhalb aufhält, und des Feststellens eines Zeitintervalls eines Aufenthalts außerhalb auszuführen, wobei die Feststellung, dass sich der Bewohner der Wohnstätte außerhalb aufhält, dadurch erfolgt, dass erkannt wurde, dass eine Eingangstür geöffnet und geschlossen wurde, und dass nach dem Schließen der Tür keine Aktivitäten mehr erkannt wurden,
wobei die Feststellung, dass der Bewohner die Wohnstätte betritt und sich somit innerhalb befindet, dadurch erfolgt, dass erkannt wurde, dass die Eingangstür nach einer Zeitdauer außerhalb geöffnet und geschlossen wurde, und Aktivitäten des Benutzers im Inneren erfasst werden,
einen Schritt des Zählens einer Anzahl von Aufenthalten außerhalb mit einem Zeitintervall, das länger als ein vorbestimmter Schwellenzeitraum ist und/oder einen Schritt des Zählens einer Anzahl von häuslichen Besuchen durch nicht in der Wohnstätte des Bewohners wohnende Besucher,
wobei die häuslichen Besuche gezählt werden, indem Erkennungsdaten der geöffneten und geschlossenen Eingangstür mit mehreren gleichzeitigen, in der Wohnstätte erfassten Aktivitäten verglichen werden,
einen Schritt des Ermittelns eines Indikators für soziale Interaktion basierend auf einer Gesamtzahl gezählter Aufenthalte außerhalb und/oder gezählter häuslicher Besuche in einem Auswertungszeitraum, und einen Schritt des Sendens eines Informationssignals an eine Softwareplattform, informierend Angehörige und/oder professionelle Betreuer, basierend auf dem ermittelten Indikator für soziale Interaktion oder einer auf einer zeitabhängigen Entwicklung des ermittelten Indikators für soziale Interaktion.

## Revendications

1. Système de surveillance (1) pour surveiller des activités humaines, comprenant un nombre multiple de capteurs de détection de présence (2a-2e) montés dans une résidence, comprenant en outre une unité de surveillance (4) connectée de manière communicante aux capteurs de détection de présence (2a-2e) et agencée pour recevoir des données de présence depuis les capteurs de détection de présence (2a-2e), dans lequel l'unité de surveillance (4) est en outre agencée pour déterminer, à partir des données de présence reçues, si l'habitant de la résidence est à l'intérieur ou à l'extérieur et pour déterminer un intervalle de temps d'une présence à l'extérieur, **caractérisé en ce que** les capteurs de détection de présence (2a-2e) incluent un ou plusieurs capteurs de porte,
dans lequel l'unité de surveillance (4) est en outre agencée pour compter un nombre de présence à l'extérieur ayant un intervalle de temps qui est plus long qu'une période de temps de seuil prédéterminée,
dans lequel l'unité de surveillance (4) est agencée pour déterminer que l'habitant de la résidence est à l'extérieur en détectant qu'une porte avant a été ouverte et fermée et que, après que la porte a été fermée, plus aucune activité n'a été détectée,
dans lequel l'unité de surveillance (4) est agencée pour déterminer que l'habitant entre dans la résidence, et est donc à l'intérieur, en détectant que la porte avant a été ouverte et fermée, après une période de temps à l'extérieur, et des activités de l'utilisateur à l'intérieur sont détectées,
dans lequel l'unité de surveillance (4) est en outre agencée pour compter un nombre de visites à domicile, par des visiteurs non résidents, dans la résidence de l'habitant en comparant des données de détection de la porte avant étant ouverte et fermée avec de multiples activités simultanées détectées dans la résidence,
dans lequel l'unité de surveillance (4) est agencée pour déterminer un indicateur d'interactions sociales en se basant sur un nombre total de présences à l'extérieur comptées et ou de visites à domicile comptées dans une période de temps d'évaluation, et
dans lequel l'unité de surveillance (4) est aussi agencée pour envoyer un signal d'information à une plate-forme logicielle informant des relations et/ou des aidants professionnels, en se basant sur l'indicateur d'interactions sociales ou sur un temps en fonction de l'indicateur d'interactions sociales déterminé.

2. Système de surveillance selon la revendication 1, dans lequel l'intervalle de temps d'une présence à l'extérieur est déterminé en détectant, à partir des données de présence reçues, un départ de l'habitant de et une arrivée de l'habitant à la résidence.

3. Système de surveillance selon la revendication 1 ou 2, dans lequel les capteurs de détection de présence incluent un seul ou plusieurs capteurs de déplacement et/ou capteurs d'occupation infrarouge.

4. Système de surveillance selon l'une quelconque des revendications précédentes, dans lequel le nombre total de présences à l'extérieur comptées sont ajoutées aux visites à domicile comptées d'une manière pondérée.

5. Système de surveillance selon l'une quelconque des revendications précédentes, dans lequel l'unité de surveillance est agencée pour déterminer l'indicateur d'interactions sociales dans des périodes de temps d'évaluations qui se suivent.

6. Procédé de surveillance d'activités humaines, comprenant les étapes de :
fournir un nombre multiple de capteurs de détection de présence (2a-2e) incluant un ou plusieurs capteurs de porte montés dans une résidence ;
fournir une unité de surveillance (4) connectée de manière communicante aux capteurs de détection de présence (2a-2e) ;
recevoir des données de présence depuis les capteurs de détection de présence (2a-2e) ;
déterminer, à partir des données présence reçues, si l'habitant de la résidence reste est à l'intérieur ou à l'extérieur et déterminer un intervalle de temps d'une présence à l'extérieur,
dans lequel la détermination que l'habitant de la résistance est à l'extérieur est réalisée en détectant qu'une porte avant a été ouverte et fermée et que, après que la porte a été fermée, plus aucune activité n'a été détectée,
dans lequel la détermination que l'habitant entre dans la résidence, et est donc à l'intérieur, est réalisée en détectant que la porte avant a été ouverte et fermée, après une période de temps à l'extérieur, et des activités de l'utilisateur à l'intérieur sont détectées,
compter un nombre de présences à l'extérieur ayant un intervalle de temps qui est plus long qu'une période de temps seuil prédéterminée, et/ou
compter un nombre de visites à domicile, par des visiteurs non résidents, dans la résidence de l'habitant en comparant des données de détection de la porte avant étant ouverte et fermé avec de multiples activités simultanées détectées dans la résidence,
déterminer un indicateur d'interactions sociales en fonction d'un nombre total de présences à l'extérieur comptées et/ou de visites à domicile comptées dans une période de temps d'évaluation, et
envoyer un signal d'information à une plate-forme logicielle informant des relations et/ou des aidants professionnels, en se basant sur l'indicateur d'interactions sociales ou sur un temps en fonction de l'indicateur d'interactions sociales déterminé.

7. Produit de programme informatique pour surveiller des activités humaines, le produit de programme informatique comprenant un code lisible informatiquement pour faire qu'un processeur réalise une étape de recevoir des données de présence depuis un nombre multiple de capteurs de détection de présence (2a-2e) incluant un ou plusieurs capteurs de porte, montés dans une résidence et connectés de manière communicante à une unité de surveillance (4), une étape pour déterminer, à partir des données de présence reçues, si l'habitant de la résistance est à l'intérieur ou à l'extérieur et pour déterminer un intervalle de temps d'une présence à l'extérieur, dans lequel la détermination que l'habitant de la résidence est à l'extérieur est réalisée en détectant qu'une porte avant a été ouverte et fermée et que, après que la porte a été fermée, plus aucune activité n'a été détectée,
dans lequel la détermination que l'habitant entre dans la résidence, et est donc à l'intérieur, est réalisée en détectant que la porte avant a été ouverte et fermée, après une période de temps à l'extérieur, et des activités de l'utilisateur à l'intérieur sont détectées,
une étape de compter un nombre de présences à l'extérieur ayant un intervalle de temps qui est plus long qu'une période de temps seuil prédéterminée, et/ou une étape de compter un nombre de visites à domicile, par des visiteurs non résidents, dans la résidence de l'habitant,
dans lequel les visites à domicile sont comptées en comparant des données de détection de la porte avant étant ouverte et fermée avec de multiples activités simultanées détectées dans la résidence,
une étape de déterminer un indicateur d'interactions sociales en fonction d'un nombre total de présences à l'extérieur comptées et/ou de visites à domicile comptées dans une période de temps d'évaluation, et une étape d'envoyer un signal d'information à une plate-forme logicielle informant des relations et/ou des aidants professionnels, en se basant sur l'indicateur d'interactions sociales ou sur un temps en fonction de l'indicateur d'interactions sociales déterminé.
